(19) Europäisches Patentamt
European Patent Office
Office européen des brevets

(11) Numéro de publication : **0 490 751 A1**

# (12) DEMANDE DE BREVET EUROPEEN

(21) Numéro de dépôt : **91403331.1**

(51) Int. Cl.$^5$ : **C07C 65/24,** C07C 69/94

(22) Date de dépôt : **09.12.91**

(30) Priorité : **14.12.90 FR 9015668**

(43) Date de publication de la demande :
**17.06.92 Bulletin 92/25**

(84) Etats contractants désignés :
**AT BE CH DE DK ES FR GB GR IT LI LU NL SE**

(71) Demandeur : **ELF ATOCHEM S.A.**
**4 & 8, Cours Michelet La Défense 10**
**F-92800 Puteaux (FR)**

(72) Inventeur : **Gillet, Jean-Philippe**
**4, rue du Garel**
**F-69530 Brignais (FR)**

(54) **Dérivés des acides naphtyloxy benzoiques et naphtoiques, leur procédé de synthèse.**

(57) La présente invention concerne les dérivés des acides (naphtyloxy) benzoïques de formule :

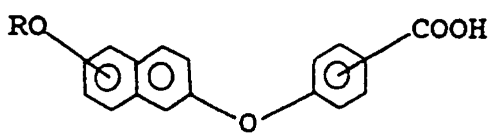

dans laquelle R représente l'hydrogène une chaîne hydrocarbonée aliphatique, cycloaliphatique ,linéaire ou ramifiée, ou un radical

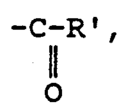

R′ étant un radical alkyle, eur procédé de synthèse et leur utilisation.

EP 0 490 751 A1

La présente invention concerne des dérivés d'acides (hydroxy, alcoxy et acétyloxy - naphtyloxy) benzoïques et naphtoïques qui répondent à l'une ou l'autre des formules :

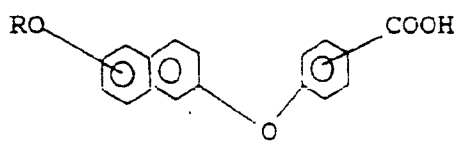

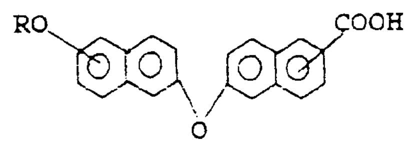

dans lesquelles R représente l'hydrogène, une chaîne hydrocarbonée aliphatique, cycloaliphatique , linéaire ou ramifiée ayant de 1 à 4 atomes de carbone ou un radical

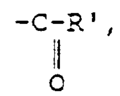

R′ étant un radical méthyle ou éthyle.

Les substituants -OR et -COOH des cycles naphtalènes des formules ci-dessus peuvent occuper les positions 1 à 8, étant entendu que les positions 1 et 3 sont particulièrement défavorables.

Parmi ces acides benzoïques, on peut tout particulièrement citer:

– les acides (méthoxy-6′ naphtyloxy-2′) 3 et 4 benzoïques
– les acides (hydroxy-6′ naphtyloxy-2′) 3 et 4 benzoïques
– les acides (acétoxy-6′ naphtyloxy-2′) 3 et 4 benzoïques

Les acides (alcoxy-naphtyloxy) benzoïques peuvent être préparés par condensation d'un dérivé halogéné d'un alcoxy naphtalène de formule:

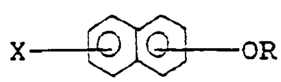

dans laquelle X est un atome d'halogène, de préférence Cl ou Br, et R répond à la définition précédente, sur l'hydroxybenzoate d'alkyle de formule

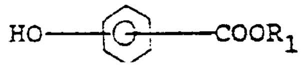

dans laquelle $R_1$ représente un radical alkyle, linéaire ou ramifié, ayant de 1 à 4 atomes de carbone, de préférence méthyle, en présence d'un composé du cuivre selon la réaction de Ullmann bien connue dans la littérature. L'ester obtenu est ensuite saponifié, par exemple à la potasse de façon à obtenir le sel de l'acide correspondant qui est acidifié.

Pour préparer les acides (alcoxy-naphtyloxy) naphtoïques on peut opérer de façon identique en remplaçant l'hydroxybenzoate d'alkyle par l'hydroxynaphtoate d'alkyle :

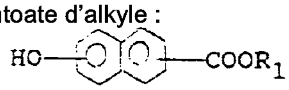

Le solvant utilisé peut être un glyme diéther de bas poids moléculaire tel que le diéthylène glycol diéthyl éther. On peut également effectuer la réaction en présence d'agent séquestrant tel qu'un éther couronne, du polyéthylène glycol(diéther) de haut poids moléculaire (≧1000), une triamine telle que définie dans la demande de brevet EP21868 de formule $N[CHR_1\text{-}CHR_2\text{-}O\text{-}(CHR_3\text{-}CHR_4\text{-}O)_n\text{-}R_5]_3$ avec $O \leqq n \leqq 10$, $R_1$, $R_2$, $R_3$, $R_4$ identiques ou différents représentant un atome d'hydrogène ou un radical alkyle de 1 à 4 atomes de carbone et $R_5$ un radical (cyclo(alkyle, phényle ou $C_mH_{2m}\varnothing$ ou $C_mH_{2m+1}\varnothing$ avec $1 \leqq m \leqq 12$ .

L'ester obtenu est ensuite saponifié et le sel de l'acide correspondant obtenu est acidifié.

Les acides (hydroxy-naphtyloxy) benzoïques peuvent être obtenus à partir des acides (alcoxy-naphtyloxy) benzoïques précédents ou de leurs sels (il n'est alors pas nécessaire de procéder à l'étape d'acidification finale décrite ci-dessus).

L'acide ou le sel est mis en solution dans l'acide acétique que l'on chauffe à une température d'environ 120°C préalablement à l'introduction de HBr. On porte l'ensemble à reflux et agite pendant plusieurs heures et laisse refroidir avant de récupérer le produit qui a précipité.

Lorsque le produit de départ est un acide, il est préférable que le rapport molaire HBr/acide soit voisin de 5.

Lorsque le produit de départ est un sel, il est nécessaire d'opérer avec un équivalent supplémentaire de HBr.

Etant donné la faible solubilité des produits de départ dans l'acide acétique, il est souhaitable d'opérer avec un fort excès d'acide acétique en général de l'ordre de 15 à 20 fois la quantité de produit de départ.

Les acides (acétoxy-naphtyloxy) benzoïques peuvent être synthétisés à partir des acides (hydroxy-naphtyloxy) benzoïques et d'anhydride d'acide.

On opère de préférence en présence d'un excès d'anhydride.

Une fois le mélange des constituants réalisé, il est chauffé jusqu'au reflux pendant 30 mn environ. On refroidit alors le mélange, le dilue à l'eau et filtre le solide obtenu. De préférence le solide est ensuite recristallisé, par exemple dans un mélange eau/acétone.

Les acides (hydroxy-naphtyloxy) et (acétoxy-naphtyloxy) naphtoïques peuvent être synthétisés de façon identique aux acides (hydroxy-naphtyloxy) et (acétoxy-naphtyloxy) benzoïques.

Les acides benzoïques et naphtoïques naphtyloxy-substitués selon l'invention peuvent être utilisés comme monomères et notamment ce sont des monomères particulièrement intéressants pour la synthèse de polymères thermotropes.

**Exemple 1**

Synthèse de l'acide (méthoxy-6′ naphtyloxy-2′)4 benzoïque

Mode opératoire

A. Dans un réacteur de 0,25 l équipé d'une bonne agitation d'un thermomètre et d'un dean stark on place:
- 0,21 M (49,8 g) de bromo-2 méthoxy-6 naphtalène
- 0,2 M (30,4 g) de parahydroxybenzoate de méthyle
- 0,2 M (27,6 g) de carbonate de potassium
- $10^{-2}$M (0,63 g) de cuivre métallique (5% molaire)
- 22 g de diéthylèneglycol diéthyléther

Le milieu est porté à 200°C sous agitation au moyen d'un bain d'huile thermostaté. On maintient 1h45 à 200°C. On distille ainsi 3,6 g de phase liquide et il se dégage 3 l de gaz. On refroidit le milieu vers 100°C et transvase dans un réacteur de 1 l puis on ajoute 0,3 M de potasse 85% dans 570cm3 d'eau. La suspension est chauffée à 100°C pendant 4h. On filtre la solution pour éliminer le catalyseur (cuivre, oxydes de cuivre).

Après séchage on obtient 57 g de sel de potassium dosé à 92 % par potentiométrie et 94 % en HPLC après acidification. Le rendement en produit isolé est de 80 %. L'acidification du filtrat et des eaux de lavage donne 2 autres fractions. Le rendement global est de 84 %.

B. Dans le même appareillage que sous A, on introduit:
- 0,308 M (73 g) de bromo-2 méthoxy-6 naphtalène
- 0,28 M (42,6g) de parahydroxybenzoate de méthyle
- 0,28 M (38,6 g) de carbonate de potassium
- 1,4 x $10^{-2}$ M (0,88 g) de cuivre métallique
- 31 g de diéthylèneglycol diéthyléther
- 1 g d'éther couronne (dicyclohexano-18 crown-6) et réalise la synthèse dans les mêmes conditions que décrites sous A. Le rendement global en sel de potassium est de 89 %.

Caractéristiques du produit

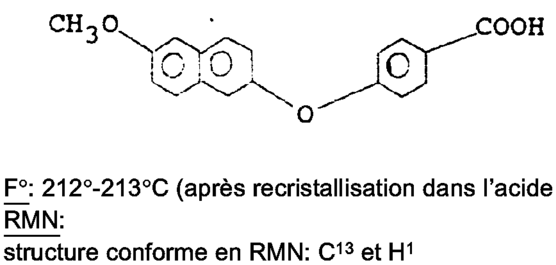

M = 294

F°: 212°-213°C (après recristallisation dans l'acide acétique).

RMN:

structure conforme en RMN: $C^{13}$ et $H^1$

pureté >99%

HPLC: un seul pic

analyse élémentaire:

| | % C | % H | % O |
|---|---|---|---|
| théorie | 73,46 | 4,79 | 21,74 |
| exp. | 73,31 | 4,53 | 21,41 |

**Exemple 2**

Synthèse de l'acide (hydroxy-6' naphtyloxy-2')4 benzoïque

**Mode opératoire**

Dans un réacteur de 1 l agité pour le reflux on place 0,275M du méthoxyacide de l'exemple 1 sous forme de sel d'acide avec 5 M (300 g) d'acide acétique puis 1,39 M (240g) d'HBr 47%. On chauffe tout d'abord la solution acétique avant de couler l'HBr en 45 mm (température ~ 120°C). Le reflux s'établit à 116°C. On laisse agiter au reflux pendant 6 h. Le bromure de méthyle qui se dégage est piégé. En fin de réaction on laisse refroidir. Le produit qui précipite est filtré et lavé à l'eau. On obtient 48,5 g de produit (pureté RMN: 95,3%). Le filtrat est dilué avec 400 cm3 d'eau pour récupérer le produit solubilisé. Le rendement total est de 78%.

Caractéristiques du produit:

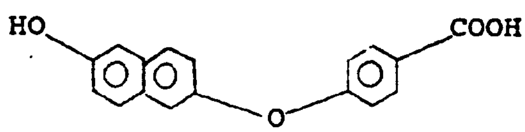

M=280

F°: 221° - 222°C
RMN:
conforme en RMN $H^1$ et $C^{13}$
pureté: 99,4% après purification
HPLC: un seul pic

**Exemple 3**

Synthèse de l'acide (acétoxy-6'naphtyloxy-2')4 benzoïque

Mode opératoire:

Dans un réacteur de 100 $cm^3$ agité équipé pour le reflux on place 20 mmoles d'hydroxyacide de l'exemple 2 dans 73 mmoles (7,5 g) d'anhydride acétique. On chauffe le tout à 136°C pendant 30 mn. On refroidit et on dilue à l'eau puis on filtre le solide.

On récupère ainsi 3,2 g après récristallisation dans un milieu acétone/eau. La pureté HPLC est de 99%.

Caractéristiques du produit:

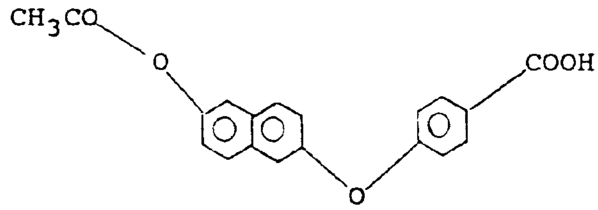

M=322

F°: 200°C
RMN:conforme en RMN $H^1$ et $C^{13}$
HPLC: un seul pic

**Exemple 4**:

Synthèse de l'acide (méthoxy-6′ naphtyloxy-2′)3 benzoïque

Mode opératoire:

Dans un réacteur équipé d'une bonne agitation, d'un thermomètre et d'un dean stark on place:
- 0,208 M (49,3 g) de bromo-2 méthoxy-6 naphtalène
- 0,19 M (29 g) de métahydroxybenzoate de méthyle
- 0,19 M (26,2 g) de carbonate de potassium
- 0,6 g de cuivre métallique
- 21,5 g de diéthylèneglycol diéthyléther

Le milieu est porté sous agitation à 200°C pendant 45′. On ajoute alors 60 g de glyme supplémentaire. On refroidit après 2h 30 de réaction. Il s'est dégagé 2, 9 l de gaz et l'on a distillé une fraction liquide de 2,6 g. On saponifie en coulant à 100°C 29 g de potasse 85 % dans 750 g d'eau. La réaction dure 4 h à 100°C. On filtre à chaud pour éliminer le cuivre. A froid le sel reste dissous. On acidifie alors avec HC1 12 N (35 cm3). Le précipité est filtré, lavé et séché: (43,7g).

pureté RMN: 97 % - Rendement : 83,7 %

On note une solubilité plus grande du sel obtenu que celle du sel de l'exemple 1.

Caractéristiques du produit

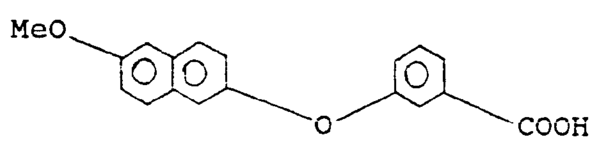

M=294

F°: 178°C
RMN :
conforme à la structure en RMN $H^1$ et $C^{13}$
pureté : 97 %
dosage potentiométrique: 97,2 %
HPLC : compatible avec le système adopté.

**Exemple 5**

Synthèse de l'acide (hydroxy-6′ naphtyloxy-2′)3 benzoïque

Mode opératoire :

Dans un réacteur de 0,5 l équipé pour le reflux on place 0,135 M (41 g) du méthoxyacide de l'exemple 4 dans 120 g (2M) d'acide acétique. Puis on coule 120 g (0,7 M) d'HBr 48 %. Le tout est porté sous agitation à

reflux (116°C) pendant 4 h puis 6 h. En fin de réaction le milieu étant homogène on précipite le composé par ajout de 600 g d'eau. On récupère ainsi un solide qui est filtré, lavé et séché (16,5 g). La pureté est de 98 % ce qui donne un rendement de 43 %.

Caractéristiques du produit

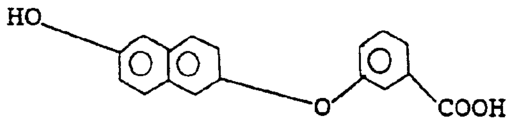

M=280

F° : 193°C - 196°C
RMN :
conforme en RMN $H^1$ et $C^{13}$
pureté : 98,8%
HPLC :
un seul pic
pureté : 98 %

**Exemple 6**

Synthèse de l'acide (acétoxy-6' naphtyloxy-2)3 benzoïque

Mode opératoire:

Dans un réacteur de 100 $cm^3$ équipé pour le reflux on place (0,02M) 5,6 g du composé de l'exemple 5 avec 16 g (0,156M) d'anhydride acétique. On porte à 130°C en agitant pendant 15 mn.

Après refroidissement, filtration, lavage à l'eau on isole 4,2 g du dérivé acétylé pur à 98,5 % Le rendement non optimisé est de 64 % en produit isolé.

Caractéristiques du produit

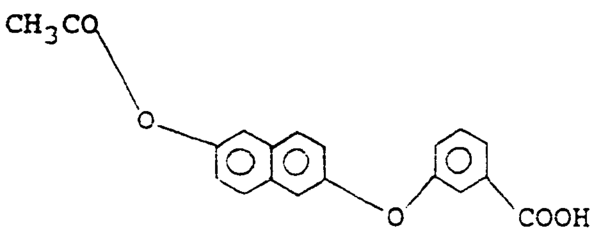

M=322

F° : 186 - 187°C
RMN :
structure conforme en RMN $H^1$ et $C^{13}$
pureté : 98,5 %
HPLC :
un seul pic dans le système adopté -
pureté : 99 %

**Revendications**

1. Dérivés des acides naphtyloxy benzoïques et naphtoïques (ou leurs sels) de formule

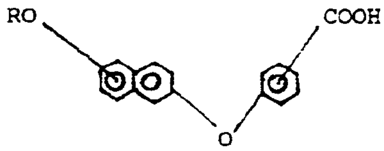

ou

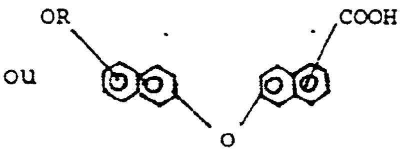

dans lesquelles R représente l'hydrogène, une chaîne hydrocarbonée aliphatique, cycloaliphatique, linéaire ou ramifiée ou un radical

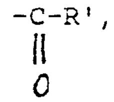

R' étant un radical alkyle.

2. Acides (méthoxy-6' naphtyloxy-2') 3 et 4 benzoïques ou leurs sels.

3. Acides (hydroxy-6' naphtyloxy-2') 3 et 4 benzoïques ou leurs sels.

4. Acides (acétoxy-6' naphtyloxy-2') 3 et 4 benzoïques ou leurs sels.

5. Procédé de synthèse des acides (alcoxy-naphtyloxy) benzoïques et naphtoïques (ou de leurs sels) tels que définis dans les revendications 1 ou 2 par condensation de Ullman en présence d'un catalyseur à base de cuivre
à partir d'un alcoxy naphtalène halogéné de formule

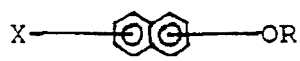

X est un atome d'halogène, et de préférence Cl ou Br, sur l'hydroxybenzoate (hydroxynaphtoate) d'alkyle suivi d'une saponification (et d'une acidification).

6. Procédé de synthèse des acides (hydroxy-naphtyloxy) benzoïques (naphtoïques) (ou de leurs sels) tels que définis dans les revendications 1 ou 3 à partir d'acides (alcoxy-naphtyloxy) benzoïques (naphtoïques) et HBr en milieu acétique.

7. Procédé de synthèse des acides (acétyloxy-naphtyloxy) benzoïques (naphtoïques) (ou de leurs sels) tels que définis dans les revendications 1 ou 4 à partir d'acides (hydroxy-naphtyloxy) benzoïques (naphtoïques) et d'anhydride d'acide.

8. Polymères obtenus à partir des acides (hydroxy-naphtyloxy) benzoïques (naphtoïques) et/ou des acides (acétoxy-naphtyloxy) benzoïques (naphtoïques) (et/ou de leurs sels).

Office européen des brevets

# RAPPORT DE RECHERCHE EUROPEENNE

Numero de la demande

EP 91 40 3331

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | US-A-3 651 134 (ELPERN ET AL.)<br>* colonne 2, ligne 40 - ligne 50; revendication 1 *<br>----- | 1 | C07C65/24<br>C07C69/94 |
| | | | **DOMAINES TECHNIQUES RECHERCHES (Int. Cl.5 )** |
| | | | C07C |

Le présent rapport a été établi pour toutes les revendications

| Lieu de la recherche | Date d'achèvement de la recherche | Examinateur |
|---|---|---|
| LA HAYE | 13 MARS 1992 | KLAG M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)